Europäisches Patentamt

European Patent Office

Office européen des brevets

Publication number: **0 367 302**

**A2**

# EUROPEAN PATENT APPLICATION

Application number: 89123550.9

Int. Cl.⁵ **C12N 9/52 , C12N 11/02 , C12P 21/02**

Date of filing: 25.04.83

This application was filed on 20 - 12 - 1989 as a divisional application to the application mentioned under INID code 60.

Priority: 23.04.82 JP 68238/82
16.07.82 JP 123917/82
18.09.82 JP 162976/82
28.09.82 JP 169428/82

Date of publication of application:
09.05.90 Bulletin 90/19

Publication number of the earlier application in accordance with Art.76 EPC: 0 092 829

Designated Contracting States:
**AT BE CH DE FR GB IT LI LU NL SE**

Applicant: **WAKO PURE CHEMICAL INDUSTRIES, LTD.**
**10, Doshomachi-3-chome**
**Higashi-ku Osaka(JP)**

Inventor: **Soejima, Masami**
**No. 847-1, Komatsu-cho**
**Tsuchiura-shi Ibaragi(JP)**
Inventor: **Suzuki, Hideya**
**No. 2120, Mochida**
**Gyoda-shi Saitama(JP)**
Inventor: **Nagaoka, Jyoji**

No. 37-3, Nishihara 2-chome
Shibuya-ku Tokyo(JP)
Inventor: **Sakata, Yoshitsugu**
No. 43-28, Uzumasa Tayabu-cho Ukyo-ku
Kyoto-shi Kyoto(JP)
Inventor: **Shintani, Akinori**
No. 43-4, Higashisonoda-cho 6-chome
Amagasaki-shi Hyogo(JP)
Inventor: **Minamii, Nobuaki**
No. 208, Hata
Neyagawa-shi Osaka(JP)
Inventor: **Matsuo, Tetsuya**
No. 22, A4-704, Yamadanishi 1-chome
Suita-shi Osaka(JP)
Inventor: **Sugiyama, Haruhiko**
No. 20-5, Onosuimei 1-chome Shiga-cho
Shiga-gun Shiga(JP)
Inventor: **Tokioka, Nobuyuki Mogawa-Ryo of
Wako Pure
Chemical Industries Ltd. No. 3-10,
Takada-cho**
**Amagasaki-shi Hyogo(JP)**

Representative: **Kolb, Helga, Dr. Dipl.-Chem. et al**
**Hoffmann, Eitle & Partner Patentanwälte**
**Arabellastrasse 4**
**D-8000 München 81(DE)**

Process for semi-synthesis of human insulin, water-soluble cross-linked Achromobacter protease I for use therein and a process for preparing the same.

A water-soluble cross-linked Achromobacter protease I with a molecular weight from 400,000 to 700,000 is described. Said enzyme is used in a process for semi-synthesizing human insulin.

EP 0 367 302 A2

## Process for semi-synthesis of human insulin, water-soluble cross-linked Achromobacter protease I for use therein and a process for preparing the same

The present invention relates to a process for semi-synthesis of human insulin and alkaline protease for use therein. More particularly, the present invention relates to a process for semi-synthesis of human insulin from porcine insulin using as a catalyst a lysyl end peptidase produced by Achromobacter lyticus and alkaline protease, in particular, immobilized or otherwise stabilized alkaline protease for use in the process.

It is all well known that insulin is a hormonal protein which is secreted from pancreas and acts an important role in reducing blood sugar and in sugar metabolism, and is essential for treatment of diabetes; animal-induced insulin is used, noting that such insulin is accompanied by side effects in human diabetes; and easy, economical and industrial production of human insulin has long been desired, etc.

Kind and sequence of amino acids which constitute insulin have already been clarified and it has long been known that only difference between human insulin and porcine insulin is that the amino acid at the C-terminal of B chain is threonine in human insulin and alanine in porcine insulin and the $B_{23}$ amino acid which is directly bound to the alanine or threonine is lysine. Some attempts to semi-synthesize human insulin from porcine insulin have been reported. It is also reported that E. coli producing human insulin was obtained utilizing genetic technology. It is recognized that these processes for preparation are epoch-making, and advanced processes as compared to conventional chemical total synthesis or partial synthesis.

A process which comprises performing a step of cutting and removing alanine at $B_{30}$ of porcine insulin to obtain des-$B_{30}$-alanine insulin, performing as a second step a step of condensing protected threonine as the amino acid at $B_{30}$ and finally obtaining human insulin is disclosed in Japanese Patent Application (OPI) Nos. 135789 79 and 138391 80 (the term "OPI" as used herein refers to a "published unexamined Japanese patent application"), Japanese Patent Publication Nos. 18798/82 and 18799/82, etc. Further, a process for substituting protected threonine at $B_{30}$ in porcine insulin in the presence of trypsin is seen in Japanese Patent Application (OPI) No. 135452 81, etc.

When examining these descriptions in detail, however, the concentration of insulins is high as compared to the amount of a reaction solvent and fluidizability of the reaction liquid essential for the reaction is extremely poor; in particular, it is quite impossible to enlarge the reaction to a scale of industrial production utilizing immobilized enzymes. Further, in these prior art processes in which very much amounts of organic solvents compared to the amount of water must be unavoidably used, dissolution of insulins is contemplated to assist by the use of organic solvents but such processes encounter disadvantage that the action of enzymes is greatly inhibited. In fact, when the same amount of organic solvents as that of water or more amounts of organic solvents are mixed with water, the efficiency of enzyme is reduced to 1 10 to 1/100 and it is compelled to use expensive enzyme in a large excess. Further, this case involved a disadvantage that enzyme not used was discarded every time.

Further E. coli producing human insulin is produced by genetic technology and it is circulated as if such would be commercially available but a question is cast thereon by the informed people (for example, Gendai Kagaku, 1981, November, page 20, left column, published by Tokyo Kagaku Dojin, Tokyo). It is no exaggeration to say that this technology is not responsible for industrial preparation, either, assuming that the E. coli obtained by genetic technology does not produce insulin as an exo-enzyme but merely produce as an endo-enzyme so that the production yield is extremely small and poorly efficient; mutants are more fragile than mother strains almost without any exception and inferior in the competition for existence.

Synthesis of human insulin from porcine insulin utilizing an enzyme having a specific action to the carboxyl group of L-lysine is described in Japanese Patent Application (OPI) No. 119085/79 by Soejima and Masaki et al., and in Biochemical and Biophysical Research Communication, Vol. 92, page 362 (1980) by Morihara and Oka et al.

According to these conventional methods, when the enzyme is reacted with porcine insulin under the condition of pH 8.5, desalanine insulin (DAI) composed of 29 amino acids in which the bond between lysine at the 29-position and alanine at the 30-position is decomposed and lysine is present on the carboxyl terminal is obtained.

Next, when DAI is reacted with threonine-OBu$^t$ under the condition of pH 6.5 in the presence of the enzyme, a new peptide bond is synthesized between lysine and threonine and human insulin-OBu$^t$ is obtained.

By hydrolysis of human insulin-OBu$^t$, human insulin can be synthesized enzymatically and chemically.

However, methods described therein are all liquid phase reactions using a water-soluble enzyme so that it is necessary that the enzyme which is a protein dissimilar to human insulin obtained be separated and purified from human insulin; if purification is insufficient, immunological side effect, i.e., formation of an antibody by a different protein is accompanied when administered to human as a medicine; in particular, when consecutively administered over long periods of time, there is a fear that an extremely serious results could occur.

Further, as a matter of course, much labors and time are required for separation and purification. Further, the enzyme after separation is inactivated and does not withstand reuse; accordingly, expensive enzyme must be discarded every preparation batch and economical loss is thus serious.

Therefore, if the enzyme is employed as a water-insoluble immobilized enzyme, it is unnecessary to perform separation and purification described above; further, it is obvious that such is advantageous from an economical viewpoint.

As methods for immobilizing enzymes, many methods are known but are roughly classified into the following five methods.

(1) a method in which an enzyme is covalently bound to a carrier;

(2) a method in which an enzyme is ionically bound to a carrier;

(3) a method in which an enzyme is adsorbed to a carrier;

(4) a method in which an enzyme is cross-linked with a cross-linking agent; and

(5) a method in which an enzyme is enclosed upon polymerization of a carrier.

In the foregoing, in the case of method (1) in which an enzyme is covalently bound to a carrier, the enzyme is bound to the carrier through a covalent bond so that binding force is strong and there is no chance that the enzyme is easily split off from the carrier. However, on the contrary, structural change in enzyme protein results in due to strong binding force and immobilized enzyme having a high activity is not obtained sometimes.

In the case of ionic binding method (2) and adsorption method (3), operations are simple but binding between the enzyme and the carrier is insufficient so that there is a drawback that the enzyme is liable to be split off from the carrier upon use.

In the case of cross-linking method (4), inactivation of the enzyme is serious and immobilizing force is weak in most cases since reaction conditions are relatively vigorous in forming the bond between the enzyme and the cross-linking agent.

In the case of enclosure method (5), a cross-linking agent or a polymerization accelerator is added to a water-soluble material, and a polymer is formed by a polymerization initiator or by irradiating light or radiations, etc., to thereby enclose the enzyme; the method involves complicated operations and inactivation of the enzyme upon enclosure is remarkable.

The enzyme is enclosed within lattices of the polymer so that enzyme protein is leaked out when the distance between the lattices becomes large.

Conversely when the lattice distance becomes too small, permeability of a substrate becomes poor so that a disadvantage that enzyme activity is reduced, etc., is involved.

As described above, any of the immobilization methods is not complete.

However, as described above, immobilization of enzymes suited for respective enzymes is desired.

It has not yet been studied to immobilize lysyl end peptidase.

It is a primary object of the present invention to provide a process for semi-synthesis of human insulin on an industrial scale.

It is another object of the present invention to provide a process for semi-synthesis of human insulin in which an enzyme used can be recovered in a stable state.

It is still another object of the present invention to provide an immobilized enzyme useful in the process for semi-synthesizing human insulin.

It is yet another object of the present invention to provide a stabilized enzyme useful for the process of semi-synthesizing human insulin.

Also, it is an object of the present invention to provide a novel alkaline protease useful for the process of semi-synthesizing human insulin.

As a result of extensive investigations on a process surely responsible for industrial production, it has been found that human insulin containing at $B_{30}$ threonine having a protective group can be obtained from porcine insulin at one step, using notably advantageous lysyl end peptidase produced by *Achromobacter lyticus* (hereinafter referred to as PLP) which has synthesis activity larger by two figures than trypsin and specifically cuts only the acid peptide bond at the carboxyl group site of lysine, as compared to trypsin which cuts all of the acid peptide bonds at the carboxyl group site of the basic amino acids present in the polypeptide chains.

Further, it has now been found that PLP can be immobilized onto a carrier without reducing its enzyme activity according to conventional methods for immobilizing enzymes.

As a result of further investigation, it has been found that PLP can be stabilized without resort to immobilization and with retaining its solubility in water, and this water-soluble enzyme can be separated and purified with ease utilizing a large difference in a molecular weight.

Another investigation has led to the discovery of a novel alkaline protease which can specifically hydrolyze the ester bond and the amide bond at the carboxyl group of L-lysine, and which is useful for the process of semi-synthesizing human insulin.

The present invention is based on the above findings.

Therefore, the present invention provides a process for semi-synthesis of human insulin by preparing human insulin derivative containing carboxyl group- protected threonine at $B_{30}$ therof from porcine insulin, characterized by using water as a reaction solvent and using lysyl end peptidase produced by Achromobacter lyticus (PLP) as a catalyst.

In another embodiment, the present invention provides an immobilized enzyme comprising PLP which is bound to a water-insoluble carrier or cross-linked with a cross-linking agent.

In still another embodiment, the present invention provides a water-soluble cross-linked polymer of PLP.

In yet another embodiment, the present invention provides a novel alkaline protease capable of catalyzing the process for semi-synthesizing human insulin from porcine insulin.

Figure 1 is a graph showing influence of pH change on amidase activity of PLP according to the present invention;

Figure 2 is a graph showing influence of pH change on esterase activity of PLP according to the present invention;

Figure 3 is a graph showing pH stability of PLP according to the present invention; In Figure 3, -o- shows the case performed using a 0.1 M calcium acetate buffer, -•- shows the case performed using 0.1 M tris-HCℓ buffer, -Δ- shows the case performed using 0.1 M diol-HCℓ buffer and -▲- shows the case performed using 0.1 M disodium phosphate-NaOH buffer;

Figure 4 is a graph showing heat stability of PLP according to the present invention;

Figure 5 is a graph showing first focusing electrophoresis-Ampholine (pH 3.5 to 10) of PLP according to the present invention;

Figure 6 is a graph showing second focusing electrophoresis-Ampholine (pH 4 to 6) of PLP according to the present invention;

Figure 7 is a graph showing pH stability of the cross-linked polymer of PLP according to the present invention in Example 11; and

Figure 8 is likewise a graph showing heat stability of the cross-linked polymer of PLP, in which solid line (—) indicates water-soluble cross-linked polymer of PLP and broken line (---) indicates PLP prior to polymerization.

## DETAILED DESCRIPTION OF THE INVENTION

Lysyl end peptidase is an exo-enzyme Achromobacter lyticus M 497-1 separated from soil produces and is the same as that found and named as Achromobacter protease I reported by Soejima and Masaki et al. in Agricultural and Biological Chemistry, Vol. 42, page 1442 (1978) (hereinafter referred to as PLP-I).

The enzyme has an optimum temperature of about 45°C; an optimum pH of 8.5 to 10.5 for protease activity when casein is used as a substrate; a molecular weight of 27,000 by a gel filtration method; an isoelectric point of pH 6.9; and is a serine enzyme which is strongly inhibited by diisopropyl phosphochloride and tosyl-L-lysine chloromethyl ketone but not inhibited by tosyl-L-phenylalanine chloromethyl ketone, ethylenediamine tetraacetate, orthophenanthroline and p-chloromercury benzoate.

The enzyme reacts with both tosyl-lysine methyl ester and tosyl-L-arginine methyl ester among synthetic substrates of esters to cause hydrolysis but the action with the latter is extremely poor and substantially negligible. Further, the enzyme has no action with an ester bond of other amino acids.

In synthetic substrates of amides, the amide bond of N-benzoyllysineamide, N-benzoyl-DL-lysine-p-nitroanilide and L-lysine-p-nitroanilide are well hydrolyzed but the corresponding arginine derivatives are not affected.

In peptides, the enzyme hydrolyzes only the amide bond between lysine at the 12-position and tyrosine at the 13-position of glucagon, only the amide bond between lysine at the 29-position and alanine at the 30-position of B chain of insulin, only the amide bond between lysine at the 8-position and glycine at the 9-position of lysine-vasopressin and only the amide bond between lysine at the 3-position and proline at the 4-position in substance P.

As such, the enzyme has a property of specifically hydrolyzing the ester bond and the amide bond in the carboxyl group of L-lysine so that the enzyme is very useful for determination of amino acid sequence in peptides or proteins.

As a result of further detailed investigations on enzyme system, particularly protease system, produced by Achromobacter lyticus. it has been found that a protease obviously different from PLP-I is present by fractionation of isoelectric point focusing electrophoresis using Ampholite (trademark of LKB Co., Ltd.) having pH 3.5 to 10. and a novel protease has been isolated from the fraction and given a name, Achromobacter protease Ia (hereinafter this novel alkaline protease is referred to as PLP-Ia).

The present inventors have extensively investigated a method for obtaining the enzyme and various properties as well as a method for utilization thereof and come to accomplish the present invention.

Thus. the present invention provides a protease having characteristics described below:

(i) Molecular weight: 30,000 (a gel filtration method using Sephadex G-75)

(ii) Isoelectric point: 5.3

(iii) pH reactivity: it has optimum pH for activity at pH 8.5 in esterase activity and has optimum pH for activity at pH 9.0 in amidase activity. respectively.

(iv) Substrate reactivity: it selectively and specifically hydrolyzes the ester bond and the amide bond at the carboxyl group of L-lysine.

(v) Inhibitor: it is inhibited by diisopropyl phosphofluoride. tosyl-L-lysine chloromethyl ketone and phenylmethylsulfonyl chloride.

Preferably a protease is produced by the genus Achromobacter.

Achromobacter lyticus M 497-1 is deposited in Foundation of Fermentation Research Institute, American Type Culture Collection and Agency of Industrial Science and Technology. Ministry of International Trade and Industry of Japan under IFO 12725, ATCC 21456 and FERM P-6718. respectively. The production of the enzyme is carried out by cultivating a bacteria producing novel protease belonging to the genus Achromobacter in a culture medium. The culture medium may be a solid or liquid. Cultivation may be subjected to settled culture but it is generally more advantageous that it is carried out under an aerobic condition using a liquid culture, such as shaking culture or aeration spinner culture, etc. To a culture medium composition. any material may be added as long as the growth of the bacteria and the production of protease Ia are accelerated. That is, as carbon sources, examples include sugars such as glucose, saccharose, dextrin, etc.; as nitrogen sources, examples include organic or inorganic nitrogen-containing compounds such as pepton, meat extract. yeast extract, dry yeast, soybean powders, casein, casamino acid, amino acids, ammonium salts, etc. As inorganic salts, metals such as sodium. potassium, calcium, magnesium, etc., may also be added in the form of phosphates. sulfates, carbonates, chlorides, etc. Depending upon cases, vitamins, nucleic acids and related compounds thereto may also be added for the purpose of accelerating the growth of the bacteria and the production of protease Ia.

Culture conditions such as liquid property of medium, culture temperature, amount of aeration, culture time, etc., vary depending upon strains used, composition of medium, etc., but, in short, are chosen and controlled appropriately such that an accumulated amount of the desired protease Ia becomes the maximum. In most cases. the liquid property of a medium is about neutral, culture temperature is between 20 and 35°C, preferably 25 and 30°C, an aeration amount is from about 0.5 to 1.5 ℓ/min per 1 liter of the medium, and culture time is about for 1 to 2 days.

Thus, a bacteria producing protease Ia is cultured and protease Ia is secreted and accumulated in the culture liquid.

To obtain the enzyme, conventional means for separation and purification can suitably be utilized and led to a standard material having optional purity. That is, the product can be fractionally precipitated by salting out through the addition of salts such as ammonium sulfate. etc., to the supernatant or the filtrate obtained after removing the cells by the process such as centrifugal separation or filtration, etc.; or by adding a hydrophilic organic solvent such as alcohols, acetone, etc., to the supernatant or the filtrate. Further, the degree of purification can be enhanced by adsorption and desorption using, e.g., alumina, bentonite, calcium phosphate gel, activated charcoal, etc., a chromatographic method using various ion exchange materials, a molecular sieve method using Sephadex, biogel, etc., singly or in suitable combination thereof. In addition, an isoelectric point precipitation method, a dialysis method, an electrophoresis method, a precipitation method using heavy metal ions, etc., can also be employed depending upon cases. As above, protease Ia having optional purity can be separated. A method for obtaining the enzyme will be explained in more detail with reference to the examples described hereafter. Titers of amidase activity and esterase activity are determined to have units, by the following methods.

Method for Measurement of Amidase Activity and Unit

5

To 1.3 mℓ of a 0.2 M 2-amino-2-methyl-1.3-propandiol buffer solution (pH 9.5), 0.15 mℓ of a 2.5 mM benzoyl-DL-lysine-p-nitroanilide (hereafter simply refer red to as Bz-Lys-p-NA) aqueous solution. After preliminarily warming to 30°C. 0.05 mℓ of an enzyme solution was added to the solution and the mixture was reacted accurately for 25 minutes. After completion of the reaction, 0.5 mℓ of a 45% (v v) acetic acid aqueous solution was added to discontinue the reaction. Then, the reaction liquid was colorimetrically measured at 405 nm and its absorbancy was determined. As an enzyme unit, an amount of the enzyme that produced 1 μmole of p-nitroaniline per 1 minute at 30°C was made 1 unit (1 u). A method for calculating enzyme titer was in accordance with the following equation.

$$\text{Activity (u/mℓ)} = \triangle OD/min \times \frac{1}{9.62} \times \frac{2.0}{0.05} \times \text{dilution magnification}$$

Method for Measurement of Esterase Activity and Unit

After preliminarily warming 3.0 mℓ of a 40 mM tris-hydrochloride buffer solution (pH 8.0) containing 1 mM tosyl-L-lysine methyl ester (simply referred to as TLME) at 30°C, 0.2 mℓ of an enzyme solution was added thereto and change of absorbancy (ΔOD) was measured at 247 nm at 30°C. An amount of the enzyme that hydrolyzed 1 μmole of TLME per 1 minute at 30°C at pH 8.0 was made 1 unit. A method for calculating an enzyme titer was in accordance with the following equation.

$$\text{Activity (u/mℓ)} = \triangle OD/min \times \frac{1}{0.96} \times \frac{3.2}{0.2} \times \text{dilution magnification}$$

Enzymatical and chemical characteristics of PLP-Ia of the present invention will next be described.

(i) Molecular weight: 30,000 (a gel filtration method using Sephadex G-75)

(ii) Isoelectric point (by an isoelectric fractionation method using Ampholine): pH 5.3

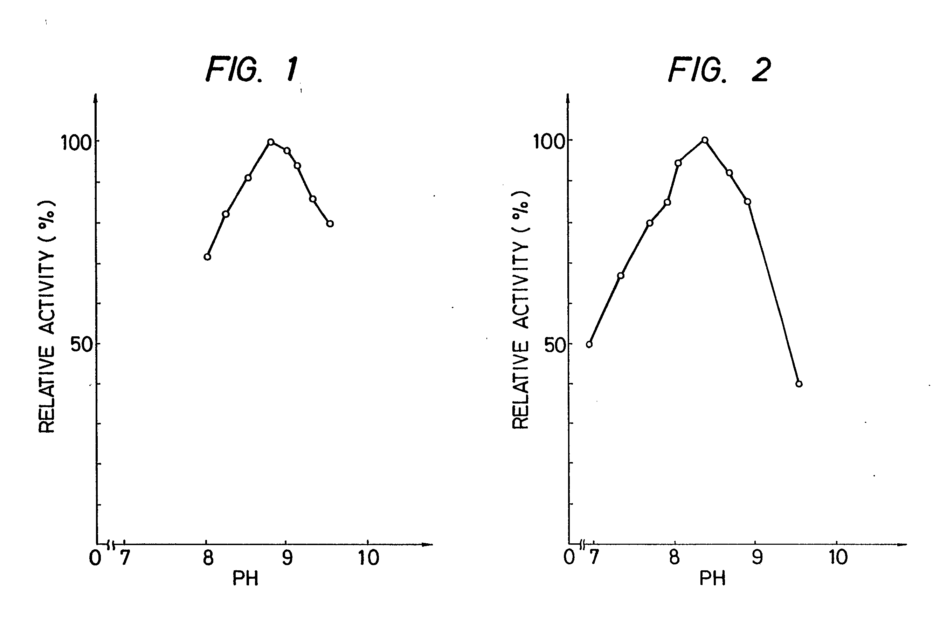

(iii) pH reactivity: it has optimum pH for activity at pH 9.0 in amidase activity (refer to Figure 1) to Bz-Lys-p-NA and has optimum pH for activity at pH 8.5 in esterase activity to TLME (refer to Figure 2).

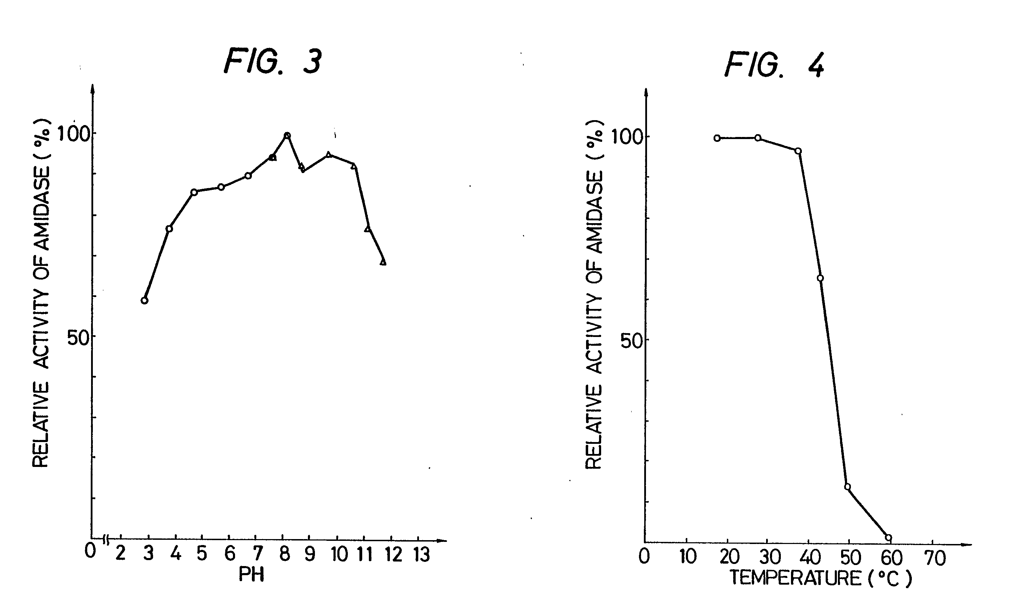

(iv) pH stability: as shown in Figure 3, it is stable over a wide pH range of from pH 5.0 to 11.0 at low temperature (treated at 4°C for 20 hours).

(v) Temperature stability: it is stable up to 40°C when heated at pH 9.0 for 15 minutes (refer to Figure 4).

(vi) Substrate reactivity: Amidase activity was measured to Bz-Lys-p-NA, N-benzoyl-L-arginine-p-nitroanilide (hereafter merely referred to as Ba-Arg-p- NA), L-lysine-p-nitroanilide (hereafter merely referred to as Lys-p-NA) and L-arginine-p-nitroanilide (hereafter merely referred to as Arg-p-NA) and esterase activity was measured to TLME and N-tosyl-L-arginine methyl ester (hereafter merely referred to as TAME), at pH 8 to 9.5 at 30°C to determine a Michaelis constant (Km) and molecular activity (Kcat) of the enzyme to each of the substrates, and the results shown in Table 1 were obtained. As is clear from this Table 1, this enzyme has an extremely high substrate specificity; the amide bond on the carboxyl group of L-lysine or L-arginine was affected in lysine but was not hydrolyzed in arginine. The enzyme hydrolyzed the ester bond of the carboxyl group in lysine or arginine; the action was strong for lysine but extremely slight for arginine.

TABLE 1

| Substrate | Quantitative Analysis | Reaction pH | Km (mM) | Kcat (sec$^{-1}$) | Kcat/Km |
|---|---|---|---|---|---|
| Bz-Lys-p-NA | A | 9.5 | 0.101 | 1.73 | 17.13 |
| Bz-Arg-p-NA | A | 9.5 | not hydrolyzed | | |
| Lys-p-NA | A | 9.0 | 0.05 | 0.04 | 0.73 |
| Arg-p-NA | A | 9.0 | not hydrolyzed | | |
| TLME | B | 8.0 | 0.09 | 523.1 | 5812 |
| TAME | C | 8.0 | 0.75 | 0.28 | 0.374 |

*Quantitative Analysis A: Method of Tuppy et al. (Z. Physiol. Chem., 329, 273 (1962))

B: Method of Schwert and Takenaka (Biochim. Biophys. Acta, 16, 570 (1955))

C: Method of Makaki et al. (Nippon Noka Shi, 51, 195 (1977))

(vii) Influence of inhibitor and various metal salts: Influences of various inhibitors on amidase activity to Bz-Lys-p-NA are shown in Table 2. Further, influences of various metal ions on amidase activity to Bz-Lys-p-NA are shown in Table 3.

TABLE 2

| Inhibitor | Concentration | Relative Activity |
|---|---|---|
| | (mM) | (%) |
| no addition (control) | -- | 100 |
| o-phenanthroline | 1 | 99.3 |
| ethylenediaminetetraacetate | 1 | 99.3 |
| L-cystein | 1 | 97.8 |
| monoiodoacetate | 1 | 84.4 |
| diisopropyl phosphofluoride | 1 | 70.1 |
| " | 10 | 53.2 |
| " | 20 | 27.5 |
| phenylmethylsulfonyl fluoride | 2 | 18.3 |
| p-chloromercury benzoate | 0.1 | 94.8 |
| tosyl-L-lysine chloromethyl ketone | 0.1 | 0 |
| tosyl-L-arginine chloromethyl ketone | 0.1 | 79.1 |
| dithiothreitol | 1 | 103.9 |
| tosyl-L-phenylalanine chloromethyl ketone | 1 | 92.2 |

As shown in Table 2, the enzyme is a sort of serine protease which undergoes inhibition by diisopropyl phosphofluoride, phenylmethylsulfonyl fluoride and tosyl-L-lysine chloromethyl ketone.

TABLE 3

| Metal Salt | Concentration | Relative Activity |
|---|---|---|
| | (mM) | (%) |
| No addition (control) | -- | 100 |
| $ZnCl_2$ | 1 | 44.1 |
| $CaCl_2$ | 1 | 102.2 |
| $NaCl_2$ | 1 | 99.0 |
| $MnCl_2$ | 1 | 101.3 |
| $NiCl_2$ | 1 | 83.8 |
| $KCl$ | 1 | 90.6 |
| $MgCl_2$ | 1 | 95.3 |
| $HgCl_2$ | 0.1 | 109.4 |
| $CoCl_2$ | 1 | 98.1 |

As is clear from Table 3, this enzyme is inhibited by zinc ions.

As described above, this enzyme has a property of specifically hydrolyzing the amide bond and the ester bond of the carboxyl group of L-lysine so that application to the fields of food chemistry, pharmaceuticals, clinical chemistry and biochemistry, etc., is expected to utilize.

Upon the use of this enzyme, a solution of this enzyme in a monomer can be used as it is; alternatively, this enzyme is cross-linked with cross-linking agents such as glutaraldehyde, diisocyanate, etc., to convert into a water-soluble or water-insoluble protease la polymer; or this enzyme is covalently bound or ionically bound to a water-insoluble carrier or enclosed therein to convert into a state insoluble in water and used as immobilized enzyme. It is needless to say that this enzyme is appropriately and advantageously employed in a mode suited for each utility or purpose. This novel enzyme has a characteristic substrate specificity that specifically acts on the peptide bond alone at the carboxyl group site of lysine and hydrolyzes the same; for this reason, this enzyme can be utilized concretely in enzyme decomposition of peptide or protein in determination of configuration and order of amino acids and decomposition and synthesis of lysyl

8

peptide.

As a specific example for utilizing this enzyme, an example of synthesizing human insulin from porcine insulin is shown. That is, the difference between porcine insulin and human insulin is that the amino acid at the 30-position is alanine in the former and threonine in the latter, other amino acid sequence is quite common therebetween and the amino acid at the 29-position adjacent the 30-position is lysine. From this, this enzyme and porcine insulin are incubated at pH 8 to 9 to cut the lysyl bond and prepare desalanine insulin (DAI) having removed alanine at the 30-position, and DAI is further condensed with threonine or a threonine derivative (for example, threonine butoxide) by this enzyme to lead to an insulin derivative. In the case of the thus-obtained insulin derivative, it is possible to finally lead to human insulin by removing the modifying group in a conventional manner.

Both PLP-I and PLP-Ia can be used in the process for semi-synthesizing human insulin according to the present invention.

The characteristic feature of the present invention lies in performing desired peptide exchange at one step by choosing water as a reaction solvent and utilizing enzyme activity of immobilized or non-immobilized PLP, and lies in also enabling one to use one or more organic solvents miscible with water in an amount the same as or larger than that of water in combination.

The peptide exchange that is characteristic of the present invention appears to occur at two steps that insulin would firstly be hydrolyzed to split alanine at $B_{30}$ off and thereafter protected threonine would be coupled; however, when the activity of enzyme is utilized under necessary conditions, that is, the enzyme reaction of the present invention is the case, direct peptide exchange is achieved by carboxyl transfer (for example. Protein, Nucleic Acid and Enzyme, Vol. 26 (1981), page 1981, right column, upper portion).

It is sufficient that the amount of water or an aqueous medium used as a reaction solvent be employed to an extent that the reaction mixture has fluidizability necessary for proceeding with the reaction or the liquid phase containing insulin, etc., easily fluidizes the layer of PLP immobilized thereto. It is unnecessary to adhere to high concentrations incapable of stirring or fluidizing, as in the prior art. The process of the present invention results in marked progress in easy procedures, smooth reaction and improvement in yield. In particular, the use of immobilized PLP provides an advantage that the loss of enzyme is minimized as less as possible.

As organic solvents used, there is no limit as long as they are miscible with water and methanol, ethanol, isopropanol, ethylene glycol, methyl cellosolve, acetone, dioxane, dimethylformamide, dimethyl sulfoxide, etc., are exemplified as normal solvents. These organic solvents are employed such that the total volume of a mixture of one or more organic solvents be in the same amount as that of water or in the amount smaller than that of water, or no organic solvent is used; in which the process of the present invention lies. Further, as a result of using organic solvents miscible with water in combination, it is unavoidable to use organic solvents that are immiscible with water in nature but become miscible with water as a whole.

The protected threonine is represented by Thr-OR. That is, the protective group is to protect the carboxyl group of threonine. It is unavoidable to protect other functional groups at the same time but in the case of using threonine having, e.g., the hydroxyl group being simultaneously protected, there is a possibility accompanying a disadvantage that requires an additional step for obtaining the desired human insulin. R in Thr-OR is substituted or unsubstituted alkyl or aralkyl and examples thereof include methyl, ethyl, propyl, isopropyl, tertiary butyl, chloroethyl, pentyl, octyl, 2-(propylsulfonyl)ethyl, benzyl, phenethyl, p-methylphenethyl, 2,4,6-trimethylbenzyl, etc.

The reaction is carried out at $50°C$ or lower but at $0°C$ or lower the reaction does not substantially proceed in a practical sense. The pH of the reaction liquid mixture is from 4 to 10, preferably about neutral.

When the range of 4 to 10 in pH is not maintained, the reaction is slow and not practical. PLP may be a soluble form or an immobilized form. That is, the reaction of the present invention may be of batch type or flow system. As immobilized PLP, those described hereinbelow can be used.

Isolation of the obtained human insulin derivative may be performed in a conventional manner; in this case, it is generally advantageous that PLP is immobilized. Further, it is performed in a conventional manner, for example, a trifluoroacetic acid method, to convert human insulin derivative having the protected threonine Thr-OR at $B_{30}$ thereof by substitution into human insulin by splitting off the protective group R.

Hereafter, immobilization of PLP according to the present invention will be described in detail. It is desired that an enzyme concentration of an aqueous solution of PLP employed in the present invention be generally adjusted to 0.01 to 30 wt%.

If the enzyme concentration is too low, an enzyme activity of the resulting immobilized enzyme is poor and such is poor for practical use.

On the contrary, when the enzyme concentration is too large, enzyme activity is saturated and economical nature becomes poor and further leakage of the enzyme occurs during the reaction.

On the other hand, as carriers employed in the present invention, polysaccharides such as cellulose, ararose, dextran, cardoran, etc., can be exemplified.

Activation of these carriers are carried out as follows using cyanogen bromide.

It is preferred that after a carrier is suspended in water and sufficiently swollen, an aqueous solution of cyanogen bromide is added to the suspension and the mixture is reacted at $40^{\circ}$ C or lower while maintaining pH alkaline using sodium hydroxide or potassium hydroxide.

The reaction time required for binding an enzyme to a carrier varies depending upon reaction temperature but is generally chosen from about 1 to 30 hours.

After completion of the reaction, the reaction product is taken out by filtration. In order to eliminate unnecessary adhesions, the reaction product is appropriately washed with a pH buffer solution or a pH buffer solution containing a salt.

Next, it has also been found that the immobilized enzyme is also obtained by a method in which PLP is ionically bound to a carrier and by a method in which PLP is cross-linked with a cross-linking agent.

The ionic binding method is a method which comprises ionically binding PLP to an ion exchange material utilizing an ionic property of the enzyme.

As carriers, there are ion exchange cellulose, ion exchange resins, etc. As a result of various investigations, however, it has been found that the immobilized enzyme is obtained by the use of cationic ion exchange resins or cationic ion exchange cellulose.

Cationic ion exchange materials are washed successively with a diluted alkali aqueous solution, water and diluted hydrochloric acid, respectively, using an about 15-fold amount. After further washing with a sufficient amount of water, the cationic ion exchange materials are taken out by filtration.

The thus-activated cationic ion exchange materials are suspended in a suitable buffer solution and a PLP solution is added to the suspension to cause ionic binding.

The reaction rapidly proceeds in a short period of time but for better yield of immobilization, it is preferred that the reaction be performed in a longer period of time and the time period is generally chosen from several minutes to 15 hours.

It is preferred that the reaction temperature in ionic binding be lower than $40^{\circ}$ C.

After completion of the reaction, the enzyme-carried product is taken out by filtration and washed appropriately with a buffer solution, etc., for removing unnecessary matters.

Also by the use of the cross-linking method, the enzyme can be immobilized.

It has been found that in addition to glutaraldehyde forming a Schiff base, immobilizing agents having a bifunctional group in the molecule thereof, i.e., isocyanic acid derivatives such as toluenediisocyanate, hexamethylenediisocyanate, hexamethylenediisothiocyanate, etc., bisdiazobenzidine, N,N'-polymethylenebismaleimide, etc., are suited for the object of the present invention.

That is, an enzyme solution is mixed with several % of a glutaraldehyde solution and the mixture is reacted while stirring.

The cross-linking agent is generally used as 1 to 5% of an aqueous solution thereof.

When a cross-linking agent of a high concentration is used, the reaction suddenly proceeds and a steric structure of an enzyme protein changes, which becomes a cause for inactivation of the enzyme.

Further in the case that the concentration of the cross-linking agent is too low, there is a drawback that the reaction becomes very slow.

As reaction conditions, the temperature is generally lower than $30^{\circ}$ C and the reaction time of 1 to 30 hours is preferably chosen.

After completion of the reaction, precipitates are centrifuged or filtered and unnecessary matters are removed using a suitable buffer solution, etc.

The thus-obtained immobilized PLP can be used immediately for enzyme reaction and can also be used appropriately depending upon necessity, by storing it in a suitable pH buffer solution at $30^{\circ}$ C or lower.

The immobilized PLP obtained in accordance with the present invention has an extremely high activity and shows high stability even over a wide pH region or in various media.

Further, the immobilized PLP can be utilized in the fields of food chemistry, medicines, clinical chemistry and biochemistry, etc., because it is non-toxic to the human and animals.

In the case of using in a batch process, the immobilized enzyme is mixed with a substrate solution. After reacting under optimum conditions, the immobilized enzyme is recovered by filtration or centrifugal separation.

The thus-recovered immobilized enzyme maintains a high activity and can be repeatedly used any time since leakage of the enzyme during the reaction is hardly observed.

On the other hand, the immobilized enzyme can also be utilized continuously by a method which comprises filling up the immobilized enzyme in a container such as a column or the like and passing a substrate solution through the container.

The immobilized enzyme has a characteristic substrate specificity that specifically acts on the peptide bond alone at the carboxyl group site of lysine and hydrolyzes the peptide bond. For this reason, the immobilized enzyme can be utilized specifically for enzymatic decomposition of a peptide in determining configuration of amino acids and decomposition of lysyl peptides as well as synthesis of lysyl peptides.

The above-described process for semi-synthesizing human insulin separates and recovers used PLP in a stable state by immobilization of the enzyme in the form of a monomer.

However, immobilized PLP becomes insoluble and thus enzyme reaction must be carried out in a suspension state, etc. It is thus a matter of course that certain device and skill must be required for smooth reaction. As contents of such device and skill, there are effective stirring for obtaining a suspension state as uniform as possible, improvement of column for prolonging a contact time, use of a relatively large amount of a suitable organic solvent in combination, use of oxygen in a large excess of amount, etc., but more or less disadvantages are unavoidable.

For further improvement, attempts have been made to stabilize PLP without relying upon immobilization to give an enzyme which is soluble in water and is capable of being separated and purified with ease utilizing a large difference in a molecular weight.

In general, when enzyme is polymerized in any process, its active site changes and its enzyme activity is decreased; however, the polymer of the present invention showed no decrease in activity and extremely unexpected, useful result has been obtained.

The water-soluble PLP cross-linked polymer of the present invention is a cross-linked polymer of PLP using a polyfunctional organic compound or the like, likewise a cross-linked polymer using polyamine as a spacer in combination, or a cross-linked polymer likewise using a water-soluble protein in combination; in any case, the resulting water-soluble cross-linked polymer of the present invention has a molecular weight of from about 400,000 to about 700,000, is soluble in water, extremely stable under environmental conditions over wide ranges and exhibits enzyme activity quite equivalent to that of PLP prior to polymerization under reaction conditions over wider ranges. Further, it is free to immobilize the polymer of the present invention to carriers suitably chosen.

In cross-linking polymerization of PLP, the final concentration of PLP in the reaction liquid is adjusted to generally 0.2 to 20 wt%, preferably 1 to 15 wt%. When the enzyme concentration becomes small, the yield of the cross-linked polymer of the present invention is decreased and practical nature is poor; when the concentration exceeds this limit, the product loses its water solubility. The thus-adjusted PLP aqueous solution is used in combination with any one of the cross-linking agents of the present invention, spacers and water-soluble proteins as shown in Table 4 to allow to be reacted. Thus, the PLP water-soluble cross-linked polymer of the present invention is obtained.

TABLE 4

| PLP | Cross-Linking Agent | Spacer | Water-Soluble Protein |
|-----|------|------|------|
| o | o | -- | -- |
| o | o | o | -- |
| o | o | -- | o |
| o | o | o | o |

As the cross-linking agents of the present invention, polyaldehydes such as glutaraldehyde, etc.; polyisocyanates such as hexamethylenedithisisocyanate, hexamethylenediisocyanate, toluenediisocyanate, etc., are generally employed, and it is preferred that the cross-linking agent be used in a concentration of about 1 to 5%. When the concentration is too high, the reaction becomes vigorous and insolubilization or the like occurs; when the concentration is too low, the yield is decreased. Examples of the spacers of the present invention include polyamines such as spermine, spermidine, etc., and, as water-soluble proteins of the present invention, proteins such as albumin, water-soluble gelatin and the like are preferred since they are supplied particularly inexpensively on an industrial scale. Any of the combinations as described in Table 4 is performed and these mixtures are incubated generally around neutral range at about 30°C for several minutes to 30 hours to cause cross-linking polymerization. Thereafter, raw materials are removed by

11

dialysis, gel filtration is performed, ultra-filtration is performed, ultracentrifugation is performed, electrophoresis using polyacrylamide gel is utilized, or, isoelectric point electrophoresis such as ampholine, etc., is utilized; in any case, the product can easily be isolated. The thus obtained PLP cross-linked polymer of the present invention can be used for enzyme reaction as it is, can be stored in a buffer solution having a suitable pH at 30°C or lower, or, can be stored as a freeze dried product; in any case, the polymer exhibits extremely effective enzyme reaction activity appropriately depending upon necessity. Further, the polymer can also be provided for immobilization thereof in a conventional manner and it is also possible to effectively use as immobilized enzyme.

The water-soluble cross-linked polymer of PLP in accordance with the present invention shows extremely high activity and specificity; further, pH stability, heat stability and stability in various media are markedly high as compared to PLP prior to polymerization. With the water-soluble cross-linked polymers obtained in Example 11 later described, the stability of these polymers is described in Tables 5, 6 and 7, in comparison with PLP prior to polymerization.

TABLE 5

| Stability in 0.1 Mole $NH_4HCO_3$ (pH 8.3) (stored at 37°C and indicated by a residual activity rate) | | | | | |
|---|---|---|---|---|---|
| | Time for Storage (hour) | | | | |
| Enzyme | 42 | 72 | 90 | 138 | 282 |
| Cross-linked polymer (%) <br> PLP prior to polymerization (%) | 93 <br> 52 | 72 <br> 33 | 69 <br> 29 | 44 <br> 17 | 14 <br> 2 |

TABLE 6

| Stability in 0.5 Mole Tris-Hydrochloride Buffer Solution (pH 6.5), Dimethylformamide and Ethanol (7 volume : 5 volume : 5 volume) (stored at 37°C and indicated by a residual activity rate) | | | | |
|---|---|---|---|---|
| | Time for Storage (hour) | | | |
| Enzyme | 24 | 42 | 90 | 234 |
| Cross-linked polymer (%) <br> PLP prior to polymerization (%) | 95 <br> 86 | 92 <br> 82 | 73 <br> 65 | 46 <br> 33 |

TABLE 7

| Stability in 0.5 Mole Acetic Acid (pH 2.5) (stored at 4°C and indicated by a residual activity rate) | | | | | |
|---|---|---|---|---|---|
| | Time for Storage (hour) | | | | |
| Enzyme | 4 | 48 | 78 | 96 | 144 | 288 |
| Cross-linked polymer (%) <br> PLP prior to polymerization (%) | 67 <br> 55 | 51 <br> 40 | 43 <br> 27 | 41 <br> 16 | 34 <br> 10 | 26 <br> 3 |

The activity of the enzyme was measured as follows. To 2.6 mℓ of 0.2 M 2-amino-2-methyl-1,3-propanediol buffer solution (pH 9.5), 0.3 mℓ of 2.5 mM benzoyllysine-p-nitroanilide aqueous solution was added. After preliminarily warming at 30°C, 0.1 mℓ of the enzyme was added to the mixture and the mixture was reacted accurately for 25 minutes at 30°C. After completion of the reaction, 1.0 mℓ of a 45% acetic acid aqueous solution was added to discontinue the reaction. Then, the reaction liquid was colorimetrically measured at wavelength of 405 nm to determine absorbance. As an enzyme unit, an amount of enzyme producing 1 μmole·min of p-nitroaniline at 30°C was made 1 unit (u). Enzyme titer was calculated according to the following equation.

$$\text{Activity (u/m}\ell) = \frac{\Delta OD}{min} \times \frac{1}{96.2} \times \frac{4.0}{0.1} \times \text{dilution}$$

Further, a liquid mixture of 0.1 mole of a liquid containing 0.144 u of the cross-linked polymer of the present invention, 0.3 mℓ of a liquid containing 2.5 mmole of each of substrates described in Table 8 and 2.6 mℓ of 0.2 M 2-amino-2-methyl-1,3-propanediol buffer solution (pH 9.5) was reacted at 30°C. Thereafter, activities to the respective substrates were measured according to the method for measuring activity described above. Results of Table 8 were obtained.

TABLE 8

| Substrate | Relative Activity (%) | |
| --- | --- | --- |
| | Cross-Linked Polymer | PLP prior to Polymerization |
| Benzoyl lysine-p-nitroanilide | 100 | 100 |
| Lysine-p-nitroanilide | 5.6 | 2.4 |
| Benzoyl arginine-p-nitroanilide | 0.04 | 0.01 |
| Leucine-p-nitroanilide | 0.00 | 0.00 |
| Alanine-p-nitroanilide | 0.00 | 0.00 |
| Glutamic acid-p-nitroanilide | 0.00 | 0.00 |
| Benzoyl tyrosine-p-nitroanilide | 0.00 | 0.00 |
| Acetylphenylalanine-p-nitroanilide | 0.00 | 0.00 |

As is evident from the table, the water-soluble cross-linked polymer of PLP in accordance with the present invention strongly acts on the bond of the carboxyl group of lysine but hardly acts on arginine and does not hydrolyze the bond of other amino acids.

In the case of using the cross-linked polymer of PLP in accordance with the present invention, the polymer is mixed with its substrate; after reacting the mixture under optimum conditions, the cross-linked polymer of the present invention is recovered by gel filtration or ion exchange materials, etc. Such an easy separation and recovery is one of advantages of the present invention and, at the same time, the cross-linked polymer of the present invention can be repeatedly used because no loss is found in its enzyme activity.

Due to such a specific activity and stability, it is a matter of course that the polymer can be utilized for enzyme decomposition of peptides in termination of amino acid configuration, decomposition and synthesis and lysyl peptides, etc., and the present inventors have established a process for semi-synthesizing human insulin using the cross-linked polymer of the present invention.

As methods for converting porcine insulin into human insulin, there are chemical methods as described in Science, Vol. 177 (1972), page 623, authored by Luttenberg, Physiological Chemistry, Vol. 357 (1976), page 759, authored by Geiger, et al., etc., and enzyme methods as described in Japanese Patent Application (OPI) Nos. 135789/79 and 18799/80, etc. The former is complicated, produces many by-products and provides low yields, and the latter is unavoidably expensive from an industrial viewpoint while some improvement was made since difficulties are seen in the amount of enzyme used and recovery of the enzyme or in the use of organic solvents.

The process of the present invention is an epoch-making process for semi-synthesizing human insulin which solves all of these drawbacks. Due to water solubility of the cross-linked polymer of PLP in accordance with the present invention, the reaction can be performed in a homogeneous system; and due to extremely high stability, it is possible to recover and repeatedly use the polymer without losing the activity of the enzyme action; or. the enzyme reaction can be carried out in high yield both in water alone or in the system in which organic solvents miscible with water are used in combination over wide ranges. Further, due to high molecular weight of the cross-linked polymer of the present invention. separation and purification of the product as well as recovery of the cross-linked polymer of the present invention are extremely advantageous since the product can easily be taken out by, for example, a molecular sieve gel filtration method.

To utilize the cross-linked polymer of the present invention for semi-synthesis of human insulin. there are two reactions. One comprises reacting porcine insulin. L-threonine in which the carboxyl group is protected or unprotected (hereafter represented by Thr-OR, wherein R is hydrogen or substituted or unsubstituted alkyl or aralkyl) and the cross-linked polymer of PLP in accordance with the present invention to prepare a human insulin derivative having Thr-OR at $B_{30}$ and then converting the derivative into human insulin in a conventional manner; the other comprises incubating porcine insulin and the cross-linked polymer of the present invention, cutting $B_{30}$ from the resulting desalanine insulin (hereafter referred to as DAI). then incubating DAI, Thr-OR and the cross-linked polymer of the present invention to prepare human insulin derivative having Thr-OR at $B_{30}$ (hereafter referred to as $B_{30}$ RO-Thr-I) and then converting the derivative into human insulin in a conventional manner.

In any case. water alone or one or more organic solvents miscible with water for increasing the solubility of raw materials in combination can be used as reaction solvents. With respect to the amount of the organic solvents used in combination. there is no particular limit. Examples of organic solvents used include methanol. ethanol. isopropanol, ethylene glycol, methyl cellosolve, acetone, dioxane. dimethylformamide. dimethyl sulfoxide, etc. The stability of the water-soluble cross-linked polymer of PLP in accordance with the present invention in these solvents is shown in Table 9 in comparison with PLP prior to polymerization, wherein there is no fear that the activity is reduced.

TABLE 9

| Stability in Solvent (stored at 30°C in solvent and indicated by a residual activity rate) | | | | | | | | |
|---|---|---|---|---|---|---|---|---|
| | Time | | | | | | | |
| | After 1 Hour | | After 24 Hours | | After 48 Hours | | After 192 Hours | |
| Solvent (examined as a 50% aqueous soln.) | A | B | A | B | A | B | A | B |
| | (%) | (%) | (%) | (%) | (%) | (%) | (%) | (%) |
| Methanol | 105 | 110 | 103 | 113 | 103 | 113 | 105 | 103 |
| Ethanol | 110 | 100 | 107 | 114 | 105 | 112 | 106 | 101 |
| Isopropanol | 104 | 99 | 107 | 114 | 99 | 110 | 103 | 101 |
| Ethylene glycol | 105 | 100 | 110 | 113 | 109 | 115 | 106 | 101 |
| Acetone | 116 | 114 | 120 | 120 | 112 | 122 | 130 | 126 |
| Dimethylformamide | 99 | 104 | 100 | 110 | 99 | 115 | 105 | 97 |
| Dimethylsulfoxide | 97 | 98 | 92 | 93 | 94 | 97 | 96 | 92 |
| Water | 100 | 112 | 98 | 110 | 92 | 106 | 95 | 88 |
| A: Cross-linked polymer of the present invention (obtained in Example 1) | | | | | | | | |
| B: PLP prior to polymerization | | | | | | | | |

Examples of R in Thr-OR include hydrogen, methyl, ethyl, isopropyl, t-butyl, benzyl, etc. The reaction temperature is below 50°C but preferably from to 40°C. The pH of the reaction system is from 4 to 10, particularly preferably 5 to 8. It is desired that the molar concentration ratio of Thr-OR to porcine insulin or DAI be large but generally sufficient to be about 1:5 to 1:1000. As buffering agents of the reaction liquid, trishydroxymethylaminomethane, citrate, phosphate buffer solutions, etc., are employed. It is preferred that the concentration of the cross-linked polymer of PLP in accordance with the present invention in the reaction liquid be about from 0.1 to 10 mg/ml. The reaction time is generally from 3 to 72 hours, in most cases, 6 to 24 hours.

14

The obtained $B_{30}RO$-Thr-I is converted into human insulin in a conventional manner ordinarily used for peptide synthesis, for example, when R is t-butyl, i.e., $B_{30}Bu^tO$-Thr-I is used, by removing t-butyl through treatment with anisole-trifluoroacetic acid. Also when R in Thr-OR is hydrogen, i.e., threonine is used, it was confirmed by an experiment using labeled threonine that the reaction of peptide exchange proceeded, but it is not easy to separate and purify the product from raw porcine insulin and such is not recommendable.

Human insulin semi-synthesized by the process of the present invention can be converted into medical preparations in a conventional manner, which are administered to patients, for example, as drugs for treating diabetes. The process of the present invention which provides human insulin extremely efficiently is industrially utilizable without any disturbance and greatly contributes to the art. Hereafter, some examples and reference examples are shown but these examples are not deemed to limit the present invention. In examples showing cross-linking polymerization, the yields are shown in polymerization one time but PLP which was not consumed in the polymerization was again subjected to subsequent cross-linking polymerization so that PLP provides the water-soluble cross-linked polymer of the present invention wastelessly without being damaged under mild conditions for polymerization.

## REFERENCE EXAMPLE 1

A liquid medium (pH 7.2) containing 1% of pepton, 0.5% of milk casein, 1.0% of saccharose, 0.01% of $K_2HPO_4$ and 0.01% of $MgSO_4 \cdot 7H_2O$ was separately charged by 100 mℓ each in a 500 mℓ volume Sakaguchi flask. After sterilization, Achromobacter lyticus M497-1 was inoculated and cultivation was performed at 28°C for 24 hours to prepare a seed culture solution. This seed culture solution, 1.5 ℓ, was transplanted to a fermentation tank having charged 30 ℓ of the same medium composition and aeration spinner culture was performed for 4 days while feeding 15 ℓ.min of air at 28°C. After cooling 30 ℓ of the obtained culture solution to about 15°C, bacteria were removed using a centrifuge to obtain about 26 ℓ of the supernatant. To the supernatant, 260 mℓ of a 4% benzalkonium chloride solution was gradually added dropwise while mildly stirring. After allowing to stand at 4°C for 1 hour, the formed precipitate was removed using a centrifuge to obtain about 25.5 ℓ of the supernatant. To the thus-obtained supernatant (4°C), 80 ℓ of acetone cooled to -5°C was gradually added while mildly stirring. After further allowing to stand in the cold overnight, the formed precipitate was collected using a centrifuge and washed with cold acetone to obtain about 40 g of wet precipitate. After drying this wet precipitate with air, the precipitate was dried for 2 days under reduced pressure in a desiccator laid with silica gel to obtain about 14 g of gray white powdery crude enzyme specimen. The amidase activity of the crude enzyme specimen to Bz-Lys-p-NA was 21.6 units/g. In a 10 mM tris-hydrochloride buffer solution (pH 8.0) 10 g of this acetone powder was dissolved and to 500 mℓ of the resulting crude enzyme solution, 200 g (wet weight) of carboxymethyl cellulose (manufactured by Brown Co., Ltd.) previously equilibrated with a 10 mM tris-hydrochloride buffer solution (pH 8.0) was added. After stirring the mixture mildly for about 1 hour, the mixture was filtered using a glass filter. The ion exchange cellulose on the filter was washed with a suitable amount of the same buffer solution and the washed liquid was combined with the previous filtrate to obtain 725 mℓ of an enzyme solution. To 725 mℓ of the obtained enzyme solution, 460 g (wet weight) of diethylaminoethyl cellulose (manufactured by Brown Co., Ltd.) previously equilibrated with the same buffer solution was added. After stirring the mixture at 4°C for 1 hour, filtration and washing were performed in a manner similar described above. The obtained filtrate was condensed using diaflow membrane UM-10. Thereafter, the condensed filtrate was dialyzed thoroughly against a 2 mM tris-hydrochloride buffer solution (pH 8.0) to obtain 492 mℓ of an enzyme solution. This enzyme solution was added and adsorbed to a column (φ 4 x 21 cm) of AH-Sepharose 4B (manufactured by Pharmacia Co., Ltd.) equilibrated with a 2 mM tris-hydrochloride buffer solution (pH 8.0). After thoroughly washing with the same buffer solution, elution was carried out with 2 ℓ of the same buffer solution in which the concentration of sodium chloride was linearly increased from 0 to 1 M. The amidase activity portions eluted at about 0.3 M to 0.5 M of the sodium chloride concentration were collected. After thoroughly dialyzing this enzyme solution with a 2 mM tris-hydrochloride buffer solution (pH 8.0), the solution was condensed using diaflow membrane UM-10 to obtain 10 mℓ of a concentrated liquid. This concentrated liquid contained protease I, in addition to protease Ia. The amidase activity of this liquid was 14.4 units/mℓ.

## EXAMPLE 1

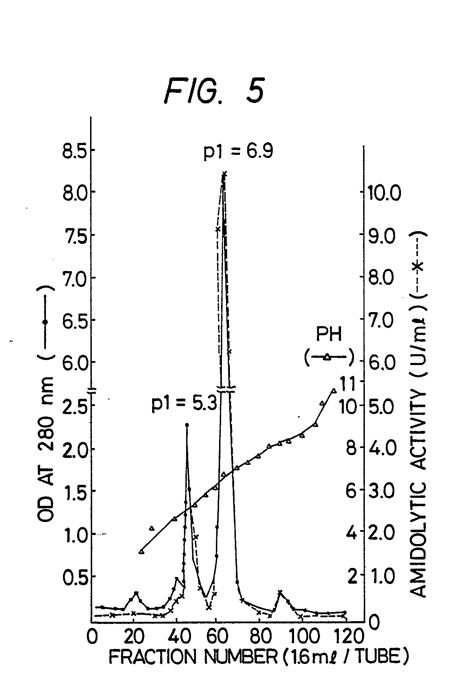

The enzyme liquid, 10 mℓ, obtained in Reference Example 1 was charged in a focusing electrophoresis device (inner volume of 150 mℓ) filled up with Ampholite (manufactured by LKB Co., Ltd.) having a pH 3.5 to 10 to subject to isoelectric point fractionation at 4°C for 48 hours at 600 v. After completion of the electrophoresis, fractionation was carried out by 1.6 mℓ each to measure the amidase activity of the respective fractions. The results as shown in Figure 5 were obtained. As is clear from Figure 5, two peaks of the amidase activity are present. Of these, the latter larger amidase activity peak corresponds to protease I. It is understood that among the peaks having the maximum amidase activity at the about 46th of the elution frac tions. i.e., two activity peaks, the former peak is not negligible. The desired enzyme is contained in this peak fraction and 12.6 mℓ of this fraction was pooled. The amidase activity was 18.9 u, specific activity (u OD280) was 12.9 and the yield was 8.7% (from acetone powder).

## EXAMPLE 2

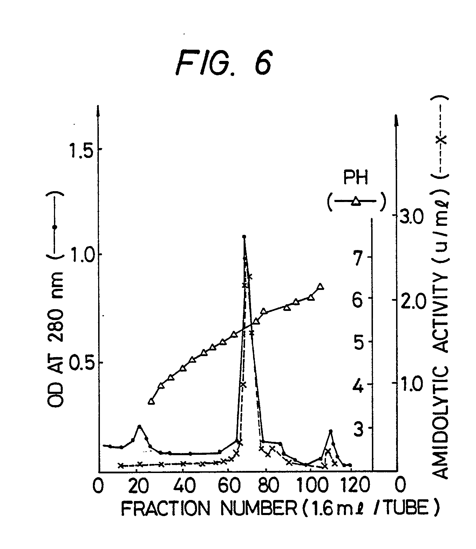

After dialyzing the enzyme solution obtained in Example 1 with a 2 mM tris-hydrochloride solution (pH 8.0), condensation was carried out. In order to remove impure proteins present still in a trace amount, electrophoresis was repeated using Ampholine (pH 4 to 6) under the same conditions as in Example 1 to obtain the results shown in Figure 6. Fractions corresponding to Fraction Numbers 65 to 76 were collected. In order to remove Ampholite co-present with this enzyme solution, the enzyme solution was passed through a column ($\phi$ 2 x 50 cm) of Sephadex G-50 equilibrated with a 2 mM tris-hydrochloride buffer solution (pH 8.0). After collecting the amidase activity fractions, the fractions were condensed to obtain 5.4 mℓ of a purified enzyme solution. The amidase activity was 16.1 units. The specific activity (u OD280) was 2.24 and the yield was 7.4% (from acetone powder). The thus-purified protease Ia of the present invention underwent electrophoresis as a single protein by analysis of a disc electrophoresis method.

This bacteria, Achromobacter lyticus M497-1 was deposited in Fermentation Research Institute, Agency of Industrial Science and Technology. The deposition number is the accession number 6718 (FERM P-6718).

## EXAMPLE 3

After neutralizing 100 mg (2 mM) of porcine insulin and 3.08 g (2 M) of L-threonine-tertiary butyl ester (Thr-OBu$^t$) with 3.5 mℓ of 5 M acetic acid, 1 M acetate buffer solution is added thereto to dissolve and the total volume was made 8.5 mℓ (pH 7.0). Thereto, 0.3 mℓ of a 1 M acetate buffer solution (pH 7.0) containing 1 mg of PLP-I was added and the mixture was kept at 37°C overnight. It is confirmed by high speed liquid chromatography that the desired compound, insulin having threonine protected with tertiary butyl at $B_{30}$ thereof [(Thr-OBu$^t$-$B_{30}$)-insulin], was formed in the yield of 65%.

After rendering the reaction liquid mixture acidic with glacial acetic acid, gel filtration was carried out using a column (40 x 200 cm) of ultra-finely divided Sephadex G-50 to fractionate an enzyme fraction, an insulin fraction and a Thr-OBu$^t$ fraction. The enzyme fraction can be reused after dialysis thereof and the Thr-OBu$^t$ fraction after condensation thereof.

After freeze drying, the insulin fraction was passed through a column (2 x 25 cm) of DEAE Sephadex A25 equilibrated with 7 M urea. After flowing the aforesaid buffer solution down at 4°C in an amount of 800 mℓ, elution was carried out with a linear concentration slope up to 3 M of the concentration of sodium chloride to obtain successively fraction A having a concentration of about 0.08 to 0.09 M and fraction B having a concentration of about 0.13 to 0.14 M. The respective fractions were immediately dialyzed to a 0.01 M ammonium acetate solution in the cold for 3 to 4 days. Thereafter, freeze drying was performed to obtain 55 mg of powders from the fraction A and 35 mg of powders from the fraction B. It is confirmed by high speed liquid chromatography and polyacrylamide gel electrophoresis that the former was [Thr-OBu$^t$-$B_{30}$]-insulin (yield 55%) and the latter was the starting material.

To 50 mg of the obtained [Thr-OBu$^t$-$B_{30}$]-insulin, 2 mℓ of trifluoroacetic acid containing 0.2 mℓ of anisole was added and the mixture was kept at room temperature for 30 minutes. Trifluoroacetic acid was removed in a nitrogen flow and anisole was extracted with 15 mℓ of ether in the presence of 2 mℓ of 1 N acetic acid. The acetic acid portion was freeze dried to obtain 43 mg of human insulin.

The product was identified to be human insulin by slab gel electrophoresis and high speed liquid chromatogram. Further, the analytical data of amino acids obtained by subjecting the product to hydrolysis with an acid (6 M hydrochloric acid was used and the reaction was at 110°C for 24 hours) are as follows and well identical with calculated values of human insulin as shown in Table 10.

TABLE 10

| | Analytical Data | | | Analytical Data | |
|---|---|---|---|---|---|
| Amino Acid | Calculated | Found | Amino Acid | Calculated | Found |
| Lys | 1 | 1.00 | His | 2 | 1.94 |
| Arg | 1 | 0.96 | Asp | 3 | 3.12 |
| Thr | 3 | 3.05 | Ser | 3 | 2.95 |
| Glu | 7 | 7.25 | Gly | 4 | 4.23 |
| Ala | 1 | 1.22 | Val | 4 | 3.86 |
| Ile | 2 | 1.89 | Leu | 6 | 6.13 |
| Thr | 4 | 3.76 | Phl | 3 | 3.12 |

In case that R of Thr-OR being tertiary butyl in the aforesaid example was replaced with methyl, isopropyl, phenethyl and p-methylphenethyl, similar course and results were obtained.


EXAMPLE 4


After neutralizing 250 mg (5 mM) of porcine insulin and 1 g (0.5 M) of Thr-OBu$^t$ with 1.5 mℓ of 5 N acetic acid, 6 mℓ of a 2 M acetate buffer solution and dimethylformamide liquid mixture in 6:4 (volume) was added thereto and pH was adjusted to 6.5. Thereto, 1.5 mℓ of a 0.2 M acetate buffer solution (pH 6.5) containing 1 mg of PLP-I was added and the mixture was kept at 37°C overnight. It can be confirmed by high speed liquid chromatography that [Thr-OBu$^t$-B$_{30}$]-insulin was formed in the yield of 75%. Then, the product was treated and purified in a manner similar to Example 3 to obtain 150 mg of human insulin. The yield was 60%.

In case that R of Thr-OR was replaced with ethyl, 2-(ethylsulfonyl)ethyl and 2,4,6-trimethylbenzyl, in place of the aforesaid example in which tertiary butyl was used, similar course and results were obtained even when the volume ratio of 2 M acetate buffer solution and dimethylformamide was changed to 5:5 and 8:2 and further the organic solvents were changed to alcohol, glycerol and dioxane.


EXAMPLE 5


After mixing 100 mg (2 mM) of porcine insulin and 3.1 g (2 M) of Thr-OBu$^t$ with 2 mℓ of 5 M acetic acid, 1 M acetic acid was added thereto to dissolve and the total volume was made 18 mℓ (pH 6.5). Thereto, PLP-I immobilized to silica gel (3 mg of PLP-I was immobilized from the enzyme activity) was added and the mixture was mildly stirred at 40°C in a nitrogen atmosphere overnight. It is confirmed by high speed liquid chromatography that [Thr-OBu$^t$-B$_{30}$]-insulin was formed in the yield of 70%.

After recovering immobilized PLP-I from the reaction liquid mixture by a centrifugal method, a 10-fold volume of acetone was added to the supernatant in the cold to precipitate insulin. After removing the solvent from a soluble portion, Thr-OBu$^t$ was recovered from the soluble portion, which is then reused. The precipitated portion was dissolved in a 0.01 M tris-buffer solution containing 7 M urea and the solution was passed through a column (2 x 25 cm) of DEAE Sephadex A25 equilibrated with the same buffer solution and [Thr-OBu$^t$-B$_{30}$]-insulin was obtained (yield 55%) and further human insulin was obtained (yield 50%) in a manner similar to Example 3.

In the case of using immobilized PLP-I using as CH-Sepharose and CH-cardoran as carriers in place of the silica gel described above in this example, the results as shown in Table 11 were obtained with the same course.

17

TABLE 11

| | | Formation Rate | Yield | |
|---|---|---|---|---|
| Carrier for Immobilized PLP-I | Enzyme Amount from Enzyme Activity | [Thr-OBu$^t$-B$_{30}$]-Insulin | [Thr-OBu$^t$-B$_{30}$]-Insulin | Human Insulin |
| | (mg) | (%) | | |
| CH-Sepharose | 3 | 78 | 60 | 55 |
| CH-Cardoran | 3 | 74 | 58 | 52 |

By the use of dimethyl sulfoxide or isopropanol in an amount the same as or smaller than that of water in combination at the step of peptide exchange in this example by adding the same to 1 M acetic acid, similar course and results were obtained.

## EXAMPLE 6

After mixing 250 mg (5 mM) of porcine insulin and 1 g (0.5 M) of Thr-OBu$^t$ with 1.5 mℓ of 5 M acetic acid, 16 mℓ of a 2 M acetate buffer solution and tetrahydrofuran liquid mixture in 7:3 (by volume) was added thereto and pH was made 6.5. Immobilized PLP-I (containing 3 mg of PLP-I from its enzyme activity) obtained by cross-linking PLP-I with glutaraldehyde was filled up in a column of 2 x 50 cm and the aforesaid liquid of pH 6.5 was circulated at room temperature in a nitrogen atmosphere. It can be confirmed by high speed liquid chromatography that [Thr-OBu$^t$-B$_{30}$]-insulin was formed in 73%. Then, human insulin was obtained in a manner similar to Example 3.

## REFERENCE EXAMPLE 2

After washing 1.3 mℓ (33 mg by dry weight) of Sepharose 4B (manufactured by Pharmacia), it was suspended in 1.3 mℓ of pure water. To the suspension 1.4 mℓ of a 2.5 w/v% cyanogen bromide aqueous solution was added. While maintaining pH at 11 with 0.1 N sodium hydroxide, the mixture was reacted at room temperature for 10 minutes. After completion of the reaction, solid matters were taken out by filtration and washed with a cold 0.1 M sodium bicarbonate aqueous solution. Then after thoroughly washing with a 0.025 M sodium borate buffer solution having pH of 8.5, activated Sepharose 4B was obtained.

## REFERENCE EXAMPLE 3

To 1.7 mℓ (34 mg on dry weight basis) of a 2% aqueous cardoran suspension, 0.7 mℓ of a 5 w/v% cyanogen bromide aqueous solution was added. An aqueous sodium hydroxide solution was added to the mixture at such a rate that pH elevated by about 0.5 per minute until the pH of the reaction mixture became 11. After the pH reached 11, the reaction was further continued for 15 minutes while maintaining the pH 11. After completion of the reaction, solid matters were taken out by filtration and thoroughly washed with pure water. Thereafter, activated cardoran gel was obtained.

## EXAMPLE 7

A 0.025 M borate buffer solution, 1.4 mℓ, of the activated Sepharose 4B prepared in Reference Example 2 was mixed with 0.9 mℓ of a 2 mM tris-hydrochloride buffer solution containing 2.68 mg of lysyl end peptidase. The reaction was performed for 1, 2, 8, 24 and 30 hours, respectively, at pH 8.5 or 10.2, while stirring at 4°C. After completion of the reaction, solid matters were taken out by filtration and washed successively with 10 ml of a sodium borate buffer solution, 10 ml of a 0.1 M sodium borate buffer solution (pH 8.5) containing 1 M sodium chloride, 10 ml of a 0.1 M sodium acetate buffer solution (pH 6.0) containing 1 M sodium chloride, 10 ml of a 0.1 M sodium bicarbonate solution containing 1.5% glycine and 20 ml of a 2 mM tris-hydrochloride buffer solution (pH 8.0) to obtain immobilized lysyl end peptidase. A rate of immobilizing proteins and activity yield of the thus obtained immobilized enzymes are shown in Table 12.

i) Measurement of Enzyme Activity

To 1.32 ml of a 0.2 M 2-amino-2-methyl-1,3-propanediol buffer solution, 0.03 ml of immobilized enzyme gel was added. After preliminarily warming the mixture at 30°C, 0.15 ml of a 2.5 mM benzoyl lysine-p-nitroanilide aqueous solution was added to the mixture and the mixture was reacted for 1 minute while stirring. After completion of the reaction, 0.5 ml of a 45% acetic acid aqueous solution was added to discontinue the reaction. Immediately thereafter, the reaction mixture was subjected to suction filtration using a glass filter and the filtrate was measured at 405 nm. 1 Unit is an amount of enzyme which forms 1 μmole of p-nitroaniline per 1 minute at 30°C.

$$\text{Activity (u/ml)} = \Delta OD/\min \times \frac{1}{9.62} \times \frac{2.0}{0.03}$$

ii) Rate of Immobilizing Protein (%)

From the amount of protein in the filtrate upon washing with water, the amount of unadsorbed protein was calculated and the amount of immobilized protein was determined.

$$\text{Rate of immobilizing protein (\%)}$$

$$= \frac{\text{Amount of protein added} - \text{Amount of unadsorbed protein}}{\text{Amount of protein added}} \times 100$$

iii) Activity Yield Immobilized (%)

It was determined by the equation:

$$\frac{\text{Activity of immobilized enzyme}}{\text{Specific activity} \times \text{Amount of immobilized protein}} \times 100$$

Here, specific activity refers to a value (u/mg) obtained by dividing the measured value for a solution of the enzyme prior to immobilization in accordance with (i) described above by the amount of protein.

TABLE 12

| pH upon Reaction | Reaction Time | Rate of Immobilizing Protein | Activity Yield Immobilized |
|---|---|---|---|
| | (hour) | (%) | (%) |
| 8.5 | 1 | 14 | 101 |
| | 8 | 32 | 86 |
| | 30 | 49 | 73 |
| 10.2 | 1 | 48.5 | 44.5 |
| | 2 | 53.5 | 44.1 |
| | 8 | 65.5 | 40.1 |
| | 24 | 79.6 | 36.8 |

In any cases that pH upon the reaction was 8.5 and 10.2, the rate of immobilizing protein increased but activity yield immobilized decreased conversely, as the reaction time became longer. In the case of reacting at pH 10.2, the rate of immobilizing protein was higher than the case of reacting at pH 8.5; however, the activity yield immobilized was conversely higher in the case of reacting at pH 8.5. This is assumed to be because the interior of the gel would become dense by binding much more enzyme to the carrier so that permeability of the substrate would become poor.

## EXAMPLE 8

A 0.025 M sodium borate buffer solution, 1.4 m$\ell$, containing the activated cardoran gel obtained by the process described in Reference Example 3 was mixed with 0.9 m$\ell$ of a 2 mM tris-hydrochloride buffer soltuion containing 2.68 mg of lysyl end peptidase and the mixture was reacted at 4°C at pH 8.5 or 10.0 for 1, 8, 24 and 30 hours, respectively, while stirring. After completion of the reaction, treatments similar to the process described in Example 7 were carried out to obtain lysyl end peptidase immobilized to cardoran. The rate of immobilizing protein and activity yield immobilized of the thus obtained immobilized enzymes are shown in Table 13.

TABLE 13

| pH upon Reaction | Reaction Time | Rate of Immobilizing Protein | Activity Yield Immobilized |
|---|---|---|---|
| | (hour) | (%) | (%) |
| 8.5 | 1 | 8.5 | 78 |
| | 8 | 17.1 | 119 |
| | 30 | 13.7 | 128 |
| 10.0 | 1 | 5.4 | 98 |
| | 8 | 7.0 | 104 |
| | 24 | 6.0 | 112 |

While the rates of immobilizing protein were lower than Reference Example 2, the activity yields immobilized were higher in any of the cases. The optimal pH of the immobilized enzymes obtained in Examples 7 and 8 were all around 10.0 to 10.5.

## EXAMPLE 9

Amberlite CG-50, 1 g (dry weight), was washed successively with 30 m ℓ of a 0.5 N sodium hydroxide aqueous solution, 30 m ℓ of water and 30 m ℓ of 0.5 N hydrochloric acid, for 10 minutes each, while stirring. After further washing with water until reaching neutral with a pH test paper, the Amberlite was thoroughly equilibrated with a 10 mM sodium acetate buffer solution having pH of 5.0. The thus-activated Amberlite CG-50 was suspended in 10 m ℓ of a 10 mM sodium acetate buffer solution (pH 5.0). To the suspension, 1 m ℓ of a 10 mM sodium acetate buffer solution (pH 5.0) containing 1 mg of lysyl end peptidase was added and the mixture was reacted for 12 hours at 4°C while stirring. After completion of the reaction, the reaction mixture was washed 5 times with 20 m ℓ of a 10 mM sodium acetate buffer solution (pH 5.0) to obtain 1.8 g (wet weight) of immobilized lysyl end peptidase (rate of immobilizing protein of 97%, activity yield immobilized, 48%).

## EXAMPLE 10

A 10 mM phosphate buffer solution (pH 7.2), 5 m ℓ, containing 10 mg of lysyl end peptidase was mixed with 5 m ℓ of a 10 mM phosphate buffer soltuion (pH 7.2) containing 4% glutaraldehyde and the mixture was reacted at 4°C for 15 hours while stirring. Thereafter, formed precipitates were subjected to centrifugal separation (8,000 rpm, 20 minutes) to separate immobilized enzyme. Unnecessary matter adhered to the immobilized enzyme were washed out with a 10 mM phosphate buffer solution having pH of 7.2 to obtain 3 mg of immobilized lysyl end peptidase (rate of immobilizing protein, 30%; activity yield immobilized, 23%).

## EXAMPLE 11

After reacting 10 mg (specific activity, 3.6 u/mg) of PLP-I and 1 m ℓ of a 0.01 M phosphate buffer solution (pH 7.2) containing 12 mg of glutaraldehyde at 4°C for 20 hours while stirring, the reaction liquid was subjected to gel filtration using a column (φ 1.5 x 87 cm) of Sephadex G-100 to obtain 17.3 units (specific activity $(u/OD_{230})$ 1.37) of a water-soluble cross-linked polymer of PLP-I in yield of 48% at the location of about 0.3 in a column volume ratio. Various properties of the product are as follows.

(i) Molecular Weight

About 450,000 (by a gel filtration method using Sephadex G-200)

(ii) Stability to pH

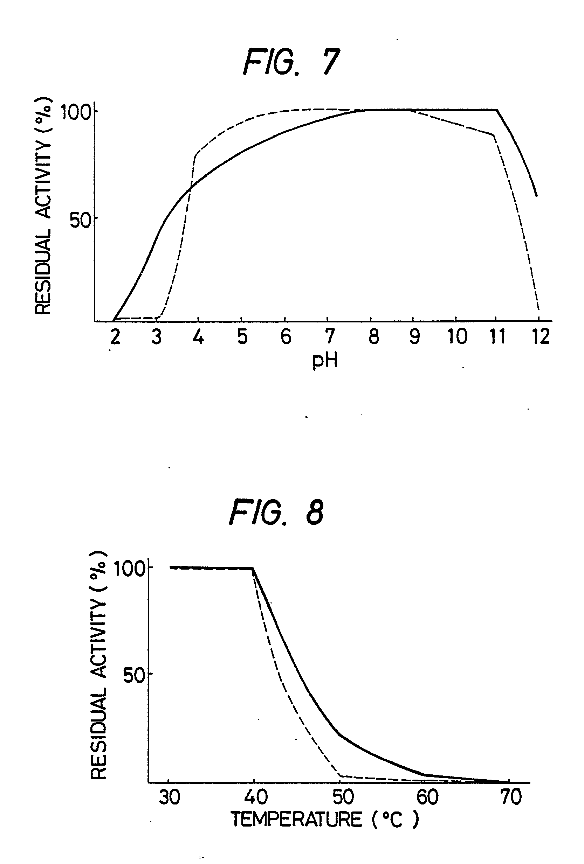

Activity was measured after maintaining at 30°C for 1 hour using a Briton-Robinson buffer solution (pH 2 to 12) as a buffer solution and the results shown in Figure 7 were obtained. From the results, it is stable at pH of 7 to 11.5.

(iii) Stability to Heat

The product was maintained for 1 hour at respective temperatures using a 0.2 M 2-amino-2-methyl-1,3-propanediol buffer solution (pH 9.5). It was stable up to 40°C as shown in Figure 8.

(iv) Michaelis Constant (Km value)

When determined at 30°C at a pH of 9.5 using benzoyllysine-p-nitroanilide as a substrate, this value was 0.15 mM.

21

(v) Optimum pH

As a result of activity measurement at 30°C at respective pH using a Briton-Robinson buffer solution (pH 2 to 12), about 9.5 was obtained.

(vi) Optimum Temperature

As a result of activity measurement at respective temperatures, it was about 40°C.

## EXAMPLE 12

After reacting 5 mg (specific activity 3.8 μ.mg) of PLP-I and 0.2 mg of spermine in 0.5 mℓ of a 0.01 M phosphate buffer solution (pH 7.2) containing 6 mg of glutaraldehyde at 4°C for 20 hours, 8.36 units (specific activity ($\mu$ $OD_{230}$) 0.797) of water-soluble cross-linked polymer of PLP-I was obtained in the yield of 44% in a manner similar to Example 11. Various properties thereof are as follows (measurement methods are the same as those in Example 11).

(i) Molecular Weight

About 500,000

(ii) Stability to pH

Stable at pH of 7 to 11.5

(iii) Stability to Heat

Stable up to 42°C

## EXAMPLE 13

After dissolving 5 mg (specific activity 3.8 μ/mg) of PLP-I and 4 mg of water-soluble gelatin (manufactured by Tanabe Pharmaceutical Co., Ltd., NP-2000) in 0.2 mℓ of a 0.01 M phosphate buffer solution (pH 7.2), 0.3 mℓ of the same buffer solution containing 6 mg of glutaraldehyde was added to the solution and the mixture was reacted at 25°C for 50 minutes. Subsequently, in a manner similar to Example 11, 5.89 units (specific activity (u/$OD_{230}$) 0.702) of a water-soluble cross-linked polymer of PLP-I was obtained in the yield of 31%. Various properties obtained according to the measurement methods described above are as follows.

(i) Molecular Weight

About 600,000 (this value is by a gel filtration method using Sepharose 6B)

(ii) Stability to pH

Stable at pH of 6 to 11

(iii) Stability to Heat

Stable up to 40°C

## EXAMPLE 14

In 0.2 mℓ of a 0.01 M phosphate buffer solution (pH 7.2), 5 mg (specific activity 2.09 u/mg) of PLP-I, 11.6 mg of bovine albumin and 0.01 mg of spermine were dissolved. After adding 0.3 mℓ of the same buffer solu tion containing 6 mg of glutaraldehyde to the solution. the mixture was reacted at 4°C for 4 hours while stirring. Thereafter in a manner similar to Example 11. 4.50 units (specific activity u/OD$_{230}$) 0.18) of a water-soluble cross-linked polymer of PLP-I were obtained in the yield of 43%. The molecular weight was measured as in Example 12 and the others were measured in a manner similar to Example 11.

(i) Molecular Weight

About 650,000

(ii) Stability to pH

Stable at pH of 4 to 11.5

(iii) Stability to Heat

Stable up to 42°C

## EXAMPLE 15

In 0.35 mℓ of a 0.01 M phosphate buffer solution (pH 7.2) 5 mg (specific activity 3.8 u/mg) of PLP-I was dissolved and 0.05 mℓ of an acetone solution containing 2 mg of hexamethylene diisocyanate and 0.1 mℓ of a 0.01 M phosphate buffer solution (pH 7.2) containing 0.2 mg of spermine were added to the solution. After reacting the mixture at room temperature for 1 hour with stirring, the procedures were performed in a manner similar to Example 11 to obtain 2.8 units (u/OD$_{230}$ 0.737) of a water-soluble cross-linked polymer of PLP-I in the yield of 14.7%. Various properties thereof are as follows.

(i) Molecular Weight

About 450,000 to 500,000 (the same method as in (i) of Example 11)

(ii) Stability to pH

Stable at pH of 7 to 11.0 (the same method as in (ii) of Example 11)

(iii) Stability to Heat

Stable up to 42°C (the same method as in (ii) of Example 11)

## EXAMPLE 16

23

Immobilization

Sepharose 4B (manufactured by Pharmacia Co., Ltd.), 0.5 g (wet weight), activated with cyanogen bromide in a conventional manner and 5.58 units of the water-soluble cross-linked polymer of PLP-I obtained in Example 11 were reacted in 2 mℓ of a 0.025 M sodium borate buffer solution (pH 8.5) at 4°C for 20 hours. The solid matter taken out by filtration was washed subsequently with a 0.025 M sodium borate buffer solution (pH 8.5), the same buffer solution (pH 8.5) containing 1M sodium chloride, a 0.1 M sodium acetate buffer solution (pH 5.0) containing 1 M sodium chloride, a 0.1 M sodium hydrogen carbonate aqueous solution contain ing 1.5% glycine and a 0.01 M tris-hydrochloride buffer solution (pH 8) to obtain 0.5 g (wet weight) of immobilized enzyme of the PLP-I cross-linked polymer (activity 0.862 unit 0.5 wet carrier).

EXAMPLE 17

Immobilization

To 2 mℓ of a 0.01 M phosphate buffer solution (pH 5.5) containing 5.34 units of the water-soluble cross-linked polymer of PLP-I obtained in Example 11, 1 g (wet weight) of Amberlite CG-50 (made by Rohm & Haas Co., Ltd.) previously activated was added to effect suspension. While stirring the suspension at 40°C, 2 mℓ of the same buffer solution containing 100 mg of 1-ethyl-3-[3-(dimethylamino)propyl]carbodiimide hydrochloride was gradually added to the suspension at 40°C with stirring. The mixture was reacted at the same temperature for further 15 hours. The solid matter taken out by filtration was washed subsequently with the same buffer solution, the same buffer solution containing 1 M sodium chloride and a 2 mM tris-hydrochloride buffer solution (pH 8.0) to obtain 1.0 g (wet weight) of immobilized enzyme of the PLP-I cross-linked polymer (activity 0.66 unit 1 g wet carrier).

EXAMPLE 18

Application to Insulin

To 100 mg (concentration 2 mM) of porcine insulin and 3.08 g (concentration 2 M) of Thr-OBu$^t$ (R = tertiary butyl in Thr-OR) neutralized with 3.5 mℓ of 5 M acetic acid, a 2 M acetate buffer solution (pH 5.5) containing 40% of an ethanol-dimethylformamide (1 vol : 1 vol) liquid mixture was added to dissolve and make the total volume 8.5 mℓ. Thereto 0.3 mℓ of a condensed of the water-soluble cross-linked polymer of PLP-I obtained in Example 11 having 10 amidase activity units mℓ liquid was added and the mixture was maintained at 37°C overnight. By confirmation with a high speed liquid chromatography, B$_{30}$Bu$^t$O-Thr-I was formed in the yield of 65%.

The reaction liquid mixture was passed through a column (φ 4 x 200 cm) of ultra-finely divided Sephadex G100 and subjected to gel filtration with a 0.5 M acetate buffer solution (pH 5.0) to fractionate into an enzyme fraction, an insulin fraction and a Thr-OBu$^t$ fraction. All can be reused by condensing the enzyme fraction or by freeze-drying the enzyme fraction followed by pulverizing, or by freeze drying the Thr-OBu$^t$ fraction. The activity recovery rate of the enzyme fraction, i.e., the water-soluble cross-linked polymer of PLP-I, was 95%.

After freeze drying, the insulin fraction was passed through a column (φ 2 x 25 cm) of DEAE Sephadex A25 equilibrated with a 7 M urea solution. After flowing 800 mℓ of the buffer solution described above down at 4°C, elution in which the concentration of sodium chloride was linearly slanted up to 0.3 M was performed to take out the 0.08 to 0.13 M fraction and the 0.17 to 0.21 M fraction. After dialyzing both fractions to a 0.01 M ammonium acetate solution, in the cold for 3 to 4 days immediately, freeze drying was performed. From the former fraction, 55 mg of B$_{30}$Bu$^t$O-Thr-I powders were obtained. The yield was 55%.

To 50 mg of the powders, 2 mℓ of trifluoroacetic acid containing 0.2 mℓ of anisole was added. After maintaining the mixture at room temperature for 30 minutes, trifluoroacetic acid was removed in a nitrogen flow and 2 mℓ of 1 M acetic acid was added. Thereafter, anisole was extracted with 15 mℓ of ether. The acetic acid portion was freeze dired to obtain 43 mg of human insulin.

The product was identified to be a standard specimen by slab gel electrophoresis and high speed liquid chromatography and confirmed to be human insulin. At the same time, the product was treated with 6 N hydrochloric acid for 24 hours at 110°C to hydrolyze and confirmed to be identical with calculated values as described in Table 14 below by amino acid analysis.

TABLE 14

| Amino Acid | Found | Calculated | Amino Acid | Found | Calculated |
|---|---|---|---|---|---|
| Lys | 1.00 | 1 | Gly | 4.01 | 4 |
| His | 1.83 | 2 | Ala | 1.07 | 1 |
| Arg | 1.01 | 1 | Val | 3.53 | 4 |
| Asp | 3.05 | 3 | Ile | 1.48 | 2 |
| Thr | 2.76 | 3 | Leu | 5.95 | 6 |
| Ser | 2.81 | 3 | Tyr | 3.77 | 4 |
| Glu | 7.13 | 7 | Phe | 3.02 | 3 |
| Pro | 1.11 | 1 | | | |

EXAMPLE 19

Application to Insulin

In 40 mℓ of 0.1 M hydrogen ammonium carbonate (pH 8.3) 200 mg of porcine insulin (concentration 0.87 mM) was dissolved and 4 mg of the water-soluble PLP-I cross-linked polymer powders obtained in Example 11 were added. The mixture was reacted at 37°C for 24 hours. The amount of alanine formed was quantitatively determined by an amino acid oxidase method and a ninhydrin method to obtain conversion rates of 98% and 92% of porcine insulin to DAI.

The freeze dried reaction liquid mixture and 6.2 g (concentration 2 M) of Thr-OBu$^t$ neutralized with 7.0 mℓ of 5 M acetic acid were dissolved in 10 mg of a 0.5 M tris buffer solution (pH 6.5) containing 40% of an ethanol-dimethylformamide (1 volume : 1 volume) liquid mixture and the solution was kept overnight at 37°C. The rate of forming $B_{30}Bu^tO$-Thr-I measured by high speed liquid chromatography was 75%. In a manner similar to Example 17, gel filtration ion exchange chromatography was performed to obtain 120 mg of $B_{30}Bu^t$-Thr-I. On the other hand, the water-soluble PLP-I cross-linked polymer was obtained in the activity recovery rate of 92%.

The obtained $B_{30}Bu^tO$-Thr-I was identified by high speed liquid chromatography and polyacrylamide gel electrophoresis.

EXAMPLE 20

Application to Insulin

In 5 mℓ of a 2 M acetate buffer solution (pH 5.1) of 3.1 g (concentration 2 M) of Thr-OBu$^t$ neutrallized with 3.5 mℓ of 5 M acetic acid 100 mg (concentration 2 mM) of porcine insulin was dissolved and 0.3 mℓ of a 10 amidase activity unit/mℓ liquid of a condensed liquid of the water-soluble PLP-I cross-linked polymer obtained in Example 11 was added to the solution and the mixture was kept at 37°C overnight. The rate of

forming $B_{30}Bu^tO$-Thr-I by high speed liquid chromatography was 60%.

Purification was carried out in a manner similar to Example 18 to obtain $B_{30}Bu^tO$-Thr-I in the yield of 50 mg. The activity recovery rate of the water-soluble PLP-I cross-linked polymer was 89%.

EXAMPLE 21

Application to Insulin

Porcine insulin, 100 mg (concentration 2 mM), and 5 mℓ of a 2 M acetate buffer (pH 5.1) solution of 3.1 g of Thr-OBu$^t$ neutralized with 3.5 mℓ of 5 M acetic acid were dissolved by adding 40% of a methanol-dimethyl sulfoxide (1 volume : 1 volume) thereto and the total volume was made 8.5 mℓ. Thereto 2 mg of the water-soluble PLP-I cross-linked polymer obtained in Example 12 was added and the mixture was kept at 37°C overnight. The reaction rate was 60%. In a manner similar to Example 18, $B_{30}Bu^tO$-Thr-I was obtained in the yield of 50 mg and the activity recovery rate of the catalyst was 93%.

EXAMPLE 22

Application to Insulin

0.3 mℓ of a 15 amidase activity unit.mℓ liquid of a condensed liquid of the water-soluble PLP-I cross-linked polymer obtained in Example 13, 100 mg (concentration 2 mM) of DAI obtained in Example 19 and 3.1 g (concentration 2 M) of Thr-OBu$^t$ neutralized with 3.5 mℓ of 5 M acetic acid were dissolved in 5 mℓ of a 0.5 M tris buffer solution (pH 6.5) containing 40% of an ethanoldimethylformamide (1 volume : 1 volume) liquid mixture and the mixture was kept at 37°C overnight. The reactivity was 68%. In a manner similar to Example 18, $B_{30}Bu^tO$-Thr-I was obtained in the yield of 55 mg and the activity recovery rate of the catalyst was 88%.

EXAMPLE 23

Application to Insulin

4.5 Units of the immobilized PLP-I cross-linked polymer obtained by the procedure of Example 16, 100 mg (concentration 2.0 mM) of the intermediate DAI obtained by the procedure of Example 19 and 3.1 g (concentration 3 M) of Thr-OBu$^t$ neutralized with 3.5 mℓ of 5 M acetic acid were mixed with 5 mℓ of a 2 M sodium acetate buffer solution (pH 7.0) containing 17% of ethanol and dimethylformamide. The mixture was kept at 37°C with stirring overnight. After filtration, the product $B_{30}Bu^tO$-Thr-I was obtained. The rate of formation by high speed liquid chromatography was 65%. The activity recovery rate of the immobilized PLP-I cross-linked polymer was 85%.

**Claims**

1. Water-soluble cross-linked Achromobacter protease I having a molecular weight of from 400,000 to 700,000.

2. The protease as claimed in claim 1, wherein said protease is cross-linked with a water-soluble protein.

3. The protease as claimed in claim 1 or 2, wherein said protease is cross-linked with a cross-linking agent together with or without the use of a spacer.

4. The protease as claimed in claims 1, 2 or 3, wherein said cross-linking agent is a polyfunctional organic compound.

5. The protease as claimed in claim 3, wherein said spacer is polyamine.

6. A process for preparing a water-soluble cross-linked Achromobacter protease I which comprises polymerizing the corresponding Achromobacter protease with a water-soluble protein together with a cross-linking agent, and further with or without the use of at least one spacer.

7. The process as claimed in claim 6, wherein said cross-linking agent is a polyfunctional organic compound.

8. The process as claimed in claim 6, wherein said spacer is polyamine.

9. The process as claimed in claims 6, 7 or 8, wherein the molecular weight of the modified enzyme is between about 400,000 and about 700,000.

10. A process for semi-synthesizing human insulin which comprises preparing a human insulin derivative having carboxyl group-protected threonine at $B_{30}$ by cutting $B_{30}$ alanine from porcine insulin and then reacting the resulting desalanine insulin with threonine having a protected carboxyl group using a water-soluble cross-linked Achromobacter protease I.

11. The process for semi-synthesizing human insulin as claimed in claim 10, wherein when said carboxyl group-protected threonine is represented by Thr-OR, R being substituted or unsubstituted alkyl or aralkyl.

*FIG. 1*

*FIG. 2*

RELATIVE ACTIVITY (%)

PH

FIG. 3

FIG. 4

EP 0 367 302 A2

FIG. 5

## FIG. 6

## FIG. 7

## FIG. 8